# EUROPÄISCHE PATENTSCHRIFT

(11) **EP 2 965 084 B1**
(45) Veröffentlichungstag und Bekanntmachung des Hinweises auf die Patenterteilung: **11.07.2018**
(21) Anmeldenummer: 14708860.3
(22) Anmeldetag: 07.03.2014
(51) Int. Cl.: G01N 33/564

(54) **VERFAHREN UND KIT ZUR ZYTOKINANALYSE AUS EINER MENSCHLICHEN VOLLBLUTPROBE**
METHOD AND KIT FOR CYTOKINE ANALYSIS FROM A HUMAN WHOLE BLOOD SAMPLE
PROCÉDÉ ET TROUSSE D'ANALYSE DES CYTOKINES D'UN ÉCHANTILLON DE SANG ENTIER HUMAIN

(30) Priorität: 08.03.2013 DE 102013204046
(43) Veröffentlichungstag der Anmeldung: 13.01.2016
(73) Patentinhaber: Universität Leipzig, 04109 Leipzig (DE)
(72) Erfinder: WAGNER, Ulf, 04179 Leipzig (DE)
(74) Vertreter: Kailuweit & Uhlemann Patentanwälte Partnerschaft mbB
(86) Internationale Anmeldenummer: PCT/EP2014/054406
(87) Internationale Veröffentlichungsnummer: WO 2014/135668

(56) Entgegenhaltungen:
- ALEX P ET AL: "Multiplex serum cytokine monitoring as a prognostic tool in rheumatoid arthritis.", CLINICAL AND EXPERIMENTAL RHEUMATOLOGY 2007 JUL-AUG, Bd. 25, Nr. 4, Juli 2007 (2007-07), Seiten 584-592, XP002722693, ISSN: 0392-856X
- HOLMGREN C ET AL: "Evaluation of the use of anti-TNF-alpha in an LPS-induced murine model", JOURNAL OF REPRODUCTIVE IMMUNOLOGY, ELSEVIER SCIENCE IRELAND LTD, IE, Bd. 78, Nr. 2, 1. Juli 2008 (2008-07-01), Seiten 134-139, XP022734112, ISSN: 0165-0378, DOI: 10.1016/J.JRI.2007.11.003 [gefunden am 2008-04-22]

## Beschreibung

### Anwendungsgebiet

Die Erfindung betrifft ein Verfahren zur prognostischen Einschätzung des Krankheitsverlaufs bei rheumatoider Arthritis, im Speziellen die prognostische Einschätzung des Krankheitsverlaufs unter Therapie, sowie zur Diagnose und/oder Aktivitätsbestimmung der rheumatoiden Arthritis durch die Analyse von Zytokinen aus einer menschlichen Vollblutprobe. Ferner betrifft die Erfindung ein dazugehörendes diagnostisches Kit und dessen Verwendung. Die Erfindung findet Anwendung in der medizinischen Diagnostik und der medizinischen Forschung.

### Stand der Technik

Rheumatoide Arthritis (RA) ist eine chronische Autoimmunerkrankung der Gelenke. Neben dem Befall der Gelenke können auch innere Organe sowie das Herz-Kreislauf-System von der Erkrankung betroffen sein. Die Ursachen der Erkrankung sind bislang weitgehend ungeklärt. Zur Diagnose wird gegenwärtig u.a. die Anwesenheit von Autoantikörpern im Serum eines Patienten bestimmt. Neben dem Nachweis des Rheumafaktors (Autoantikörpern, die gegen körpereigene IgG-Moleküle gerichtet sind) kann durch den Nachweis von anti-CCP-Antikörpern, die gegen citrullinierte Seitenketten von Proteinen gerichtet sind, eine frühere Diagnose der RA getroffen werden (Schellekens 1998). Eine vergleichbar gute Sensitivität ist durch den Nachweis des mutierten citrullinierten Vimentins, MCV, möglich (Poulsom 2008). Sowohl für den Nachweis der anti-CCP Antikörper als auch der anti-MCV Antikörper sind bereits kommerzielle Testkits erhältlich, die einen einfachen und schnellen Nachweis der Antikörper und damit eine Aussage zur Diagnose von RA ermöglichen. Mit dem Nachweis der systemisch im Blut vorhandenen Autoantikörper ist jedoch eine Einschätzung des Stadiums der Erkrankung, insbesondere des Status der Gelenksdestruktion, nur sehr bedingt möglich.

Es ist daher wünschenswert, eine Diagnostik der RA einzusetzen, bei der der Aktivierungsstatus der an der Erkrankung beteiligten Immunzellen berücksichtigt wird. Es ist bekannt, dass bestimmte Zellpopulationen der Immunzellen, wie beispielsweise T-Zellen oder Monozyten, je nach Krankheitsstadium der RA unterschiedliche Zytokine sezernieren, die eine Aussage über das Fortschreiten der RA erlauben. Es ist auch bekannt, dass diese Zellen zu einem geringen Anteil auch im Blutkreislauf des Patienten zirkulieren.

Alex P et al. 2007 beschreiben ein Zytokine-Profiling in Serumproben von an rheumatoider Arthritis erkrankten Patienten. Mittels eines biometrischen Multiplex-Assay werden die Zytokine IL-1β, IL-2, IL-7, Il12p40, Il-17, TNF-α, G-CSF, GM-CSF, IL-4, Il-5, Il-6, IL-10, IL,13, CCL2 (MCP-1), CCL3 (MIP-1α) und CXCL8 (IL-8) untersucht. Eine ungewollte Stimulation wird explizit vermieden, in dem die Blutproben in Endotoxin-freien, Silikon-beschichteten Röhrchen ohne Zusätze gesammelt werden.

Holmgren C et al. 2008 untersuchen den Effekt einer anti-TNF-α Verabreichung auf die durch LPS hervorgerufene Inflammation und deren Auswirkungen auf die Schwangerschaft und die Frühgeburtsrate in einem Mausmodel.

Es wurde beobachtet, dass durch eine Stimulation geeigneter Signaltransduktionswege von Zellen des Immunsystems bei RA unterschiedliche Zytokinspiegel in RA-Patienten im Vergleich zu gesunden Kontrollen feststellbar sind. Meusch 2009 beschreiben, dass die Signalgebung über transmembranes TNF-α (Tumornekrosefaktor, hierin wird die noch gebräuchliche Abkürzung "TNF-α" verwendet) in Monozyten aus dem Blut von RA-Patienten mit einer vermehrten Bildung von IL-1β assoziiert ist. Dazu werden Monozyten zunächst aus dem Blut der Patienten isoliert, ausgezählt und anschließend in einer definierte Anzahl in Zellkulturmedium in Gegenwart von anti-TNF-α kultiviert, wobei im Anschluss mittels ELISA die Spiegel von IL-1β bestimmt wurden.

Es ist ferner wünschenswert, mittels einfacher Testverfahren eine prognostische Aussage hinsichtlich des Krankheitsverlaufs treffen zu können. Besonders gewünscht ist dabei eine Prognose hinsichtlich des Ansprechens eines Patienten auf eine spezielle Therapieform.

Für einen klinischen Einsatz ist es dabei erstrebenswert, möglichst einfach zu handhabende Testsysteme und entsprechende Verfahren bereitzustellen, mit denen der jeweils gewünschte Nachweis in möglichst wenigen Verfahrensschritten, mit geringem Materialaufwand und dennoch mit hoher Genauigkeit durchgeführt werden kann.

### Aufgabe

Aufgabe der Erfindung ist es daher, ein Verfahren und ein Testsystem zur Analyse der Produktion proinflammatorischer Zytokine von Immunzellen aus Proben von potentiellen Patienten mit rheumatoider Arthritis bereitzustellen, mit dem eine prognostische Aussage zum Krankheitsverlauf, im Speziellen die prognostische Einschätzung des Krankheitsverlaufs unter Therapie bzw. eine Diagnose möglich ist. Das Verfahren soll dabei einfach und reproduzierbar sein und eine möglichst frühzeitige Diagnose bzw. prognostische Einschätzung ermöglichen.

### Lösung

Die Aufgabe wird erfindungsgemäß gelöst durch ein Verfahren gemäß Anspruch 1 zur Diagnose oder prognostischen Einschätzung des Krankheitsverlaufs bei rheumatoider Arthritis, insbesondere des Krankheitsverlaufs unter Therapie (speziell unter Therapie mit einem TNF-inhibierenden Arzneimittel), bei dem eine Zytokinanalyse aus einer menschlichen Vollblutprobe erfolgt. In dem erfindungsgemäßen Verfahren werden folgende Verfahrensschritte durchgeführt:
- Ein Volumen einer Vollblutprobe eines Menschen wird in mindestens ein Röhrchen mit einem stimulierenden Agens überführt. Bevorzugt wird die Vollblutprobe unmittelbar nach der Entnahme in das Röhrchen mit dem stimulierenden Agens überführt. Das eingesetzte Volumen der Vollblutprobe beträgt vorzugsweise 3 - 8 ml, bevorzugt 3 - 5 ml. Als Vergleichsproben wird jeweils das gleiche Volumen einer Vollblutprobe des Menschen in jeweils ein Leerröhrchen als Negativkontrolle und ein Röhrchen enthaltend Lipopolysaccharid als Positivkontrolle überführt.
- Die jeweiligen Röhrchen mit den darin enthaltenen Vollblutproben (Positivkontrolle, Negativkontrolle sowie das mindestens eine Röhrchen mit dem stimulierenden Agens) werden anschließend, vorzugsweise für mehrere Stunden, vorzugsweise 4 - 48 h, besonders bevorzugt 12 - 24 h, bei einer Temperatur von 30 - 40 °C, vorzugsweise 36 - 38 °C, inkubiert.
- Anschließend wird aus den jeweiligen Röhrchen der zellfreie Überstand der jeweiligen Vollblutprobe gewonnen. Aus dem zellfreien Überstand wird die Konzentration mindestens eines proinflammatorischen Zytokins bestimmt. Es wird dabei die Konzentration mindestens eines der folgenden Zytokine ermittelt: TNF-α, IL-8, IFNγ, IL-10, IL-1β und IL-6. Ebenfalls möglich ist die Konzentrationsbestimmung von einem oder mehreren gelösten Rezeptoren, bevorzugt ausgewählt aus TNFR1, TNFR2, IL1R Antagonist, IL-1R1, IL-1R2.
- Anhand einer veränderten Konzentration des mindestens einen Zytokins in dem mindestens einen Röhrchen mit dem stimulierenden Agens im Vergleich zur Negativkontrolle erfolgt anschließend die prognostische Einschätzung des Krankheitsverlaufs der rheumatoiden Arthritis, insbesondere die prognostische Einschätzung des Krankheitsverlaufs von rheumatoider Arthritis bei Therapie mit einem TNF-blockierenden Arzneimittel (der sogenannten "Therapieresponse").

Alternativ oder in Kombination zur prognostischen Einschätzung des Krankheitsverlaufs der rheumatoiden Arthritis, insbesondere der prognostischen Einschätzung der Therapieresponse der rheumatoiden Arthritis auf TNF-blockierende Arzneimittel (Tumornekrosefaktor-blockierende Arzneimittel, früher "Tumornekrosefaktor-alpha"-blockierende Arzneimittel), wird mit der Erfindung anhand der veränderten Konzentration der Zytokine im Röhrchen mit dem stimulierenden Agens die Diagnose der rheumatoiden Arthris getroffen. Alternativ oder in Kombination dazu wird mit der Erfindung eine prognostische Aussage zur Schwere einer Gelenksdestruktion im Rahmen der rheumatoiden Arthritis getroffen.

Mit der Erfindung wird ein Verfahren angegeben, mit dem es möglich ist, eine Prognose des Krankheitsverlaufs der rheumatoiden Arthritis und/oder die Diagnose der rheumatoiden Arthritis anhand der Konzentration der Zytokine zu treffen, die von Zellen des peripheren Bluts eines Menschen sezerniert werden. Besonders überraschend ist dabei, dass es möglich ist, die Analyse unter Verwendung einer menschlichen Vollblutprobe durchzuführen und dass keine weitere Isolation diskreter Zellpopulationen (beispielsweise Monozyten oder T-Zellen) aus der Vollblutprobe erforderlich ist. Da die Zellen, die die nachzuweisenden Zytokine sezernieren, insbesondere Monozyten und T-Zellen, im Vollblut in geringen Konzentrationen enthalten sind, war es äußerst überraschend, dass nachweisbare Zytokinspiegel bei Durchführung eines erfindungsgemäßen Verfahrens im zellfreien Überstand (also dem mit Zytokinen angereicherten Plasma) enthalten sind. Damit ist es möglich, den zellfreien Überstand nach der Inkubation der Vollblutproben aus jeweils einem einzigen Röhrchen zu gewinnen, ohne dass die jeweils getestete Vollblutprobe im Verfahren zwischen mehreren Gefäßen transferiert werden muss (sogenanntes "in tube" Verfahren). Das Verfahren ist damit äußerst einfach und mit einem geringen Materialaufwand realisierbar.

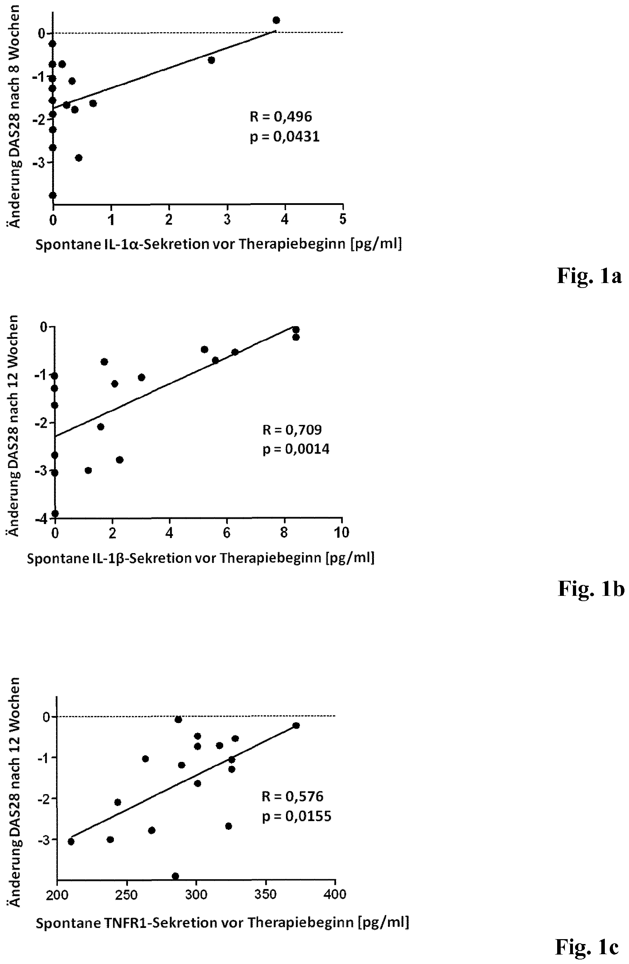

Es ist ferner überraschend, dass anhand einer veränderten Zytokinproduktion von Zellen des peripheren Bluts eine prognostische Einschätzung des Krankheitsverlaufs, insbesondere eine Prognose einer Therapieresponse bei rheumatoider Arthritis, erfolgen kann. Das erfindungsgemäße Verfahren eignet sich dabei insbesondere für die Prognose des Ansprechens des Patienten auf eine therapeutischer Behandlung mit einem TNF-inhibierenden Arzneimittel (Fig. 1 bis 7). Überraschend konnten die Erfinder demonstrieren, dass eine veränderte Zytokinproduktion bei spezifischer Stimulation diskreter Signaltransduktionswege eine Assoziation mit dem Krankheitsverlauf nach Therapie mit einem TNF-Inhibitor zeigt.

Gemäß der Erfindung kommt im erfindungsgemäßen Verfahren eine Vollblutprobe zum Einsatz, also eine Blutprobe eines Menschen. Diese Vollblutprobe wird vorzugsweise mit dem Gerinnungshemmer Heparin versetzt. Eine weitere Aufreinigung und Isolation diskreter Zellpopulationen ist nicht notwendig. Die Vollblutprobe umfasst daher sämtliche natürlicherweise im Blut vorhandenen Zellen und Plasma. Insbesondere sind folgende Zellen in der Vollblutprobe enthalten: Erythrozyten, Thrombozyten und Leukozyten, nämlich neutrophile, eosinophile und basophile Granulozyten, Lymphozyten und Monozyten. Die Vollblutprobe wird vorzugsweise unmittelbar nach der Entnahme eingesetzt, bevorzugt maximal 60 Minuten, vorzugsweise maximal 30 Minuten nach Entnahme aus dem potentiellen Patienten.

Bei denen für die Erfindung eingesetzten Röhrchen handelt es sich um für die Zellkultur geeignete Röhrchen, vorzugsweise Röhrchen aus einem inerten Kunststoff, bevorzugt Polystyrol, die eine hydrophile Oberfläche aufweisen. Das Volumen eines Röhrchens beträgt vorzugsweise 5 - 15 ml, vorzugsweise 5 - 10 ml. Auf der Oberfläche des inerten Kunststoffs befindet sich auf der Innenseite des Röhrchens vorzugsweise eine Beschichtung mit einem bioaktiven Protein, vorzugsweise Kollagen, Fibrinogen oder Fibronektin, um günstige Zellkulturbedingungen zu schaffen. Als Negativkontrolle wird ein sogenanntes Leerröhrchen eingesetzt. Unter dem Leerröhrchen im Sinne der Erfindung wird ein für die Zellkultur geeignetes Röhrchen, bevorzugt der vorstehend angegebenen Dimensionen und vorzugsweise mit einer vorstehend beschriebenen Beschichtung, verstanden, welches keine weiteren Agenzien, insbesondere keine Agenzien enthält, die die Zytokinproduktion von Zellen des Immunsystems anregen. Als Positivkontrolle wird ein Röhrchen eingesetzt, welches bakterielle Lipopolysaccharide (LPS) enthält. Diese liegen entweder als Bestandteil einer Beschichtung auf der inneren Seite des Röhrchens vor oder als Feststoff oder wässrige Lösung innerhalb des Röhrchens. Im Röhrchen ist dabei vorzugsweise so viel LPS enthalten, dass nach Überführung des definierten Volumens der Vollblutprobe eine Konzentration von 80 - 120 ng/ml LPS in der Flüssigkeit erreicht wird. Bevorzugt ist in dem Röhrchen für die Positivkontrolle 200 - 900 ng LPS enthalten.

Besonders bevorzugt sind die Röhrchen, also sowohl für die Positivkontrolle, Negativkontrolle als auch das mindestens eine Röhrchen mit einem stimulierenden Agens, als Blutentnahmeröhrchen ausgestaltet. Dies erlaubt die direkte Entnahme der Vollblutprobe in das Teströhrchen. Davon besonders bevorzugt ist die Ausgestaltung des Blutentnahmeröhrchens nach dem Unterdrucksystem. Dabei herrscht im Röhrchen von vornherein ein Unterdruck, wodurch das Blut angesaugt wird. Mit dem Unterdrucksystem können reproduzierbarere Volumina bei der Blutentnahme in das Röhrchen überführt werden, als bei der Blutentnahme nach dem Aspirationssystem (bei dem ein Unterdruck durch Herausziehen eines Stempels bei der Blutentnahme erzeugt wird). Alternativ können mit den Röhrchen auch Vollblutproben definierter Volumina aus bereits entnommenem Vollblut überführt werden (beispielsweise aus einem Blutbeutel).

In einem der Teströhrchen befindet sich ein sogenanntes "stimulierendes Agens", welches zur Aktivierung eines diskreten Signaltransduktionsweges in einer Zelle des Immunsystems geeignet ist, wobei bei Aktivierung des Signaltransduktionsweges die Sezernierung von Zytokinen durch die jeweilige Zelle beeinflusst wird. Bei dem stimulierenden Agens handelt es sich hingegen nicht um Substanzen, die eine Vielzahl von Signaltransduktionswegen unspezifisch anregen. Insbesondere ist das stimulierende Agens kein TLR-Ligand, insbesondere kein LPS. Erfindungsgemäß ist das stimulierende Agens ausgewählt aus einem spezifischer Bindungspartner von tmTNF-α (bevorzugt anti-TNF-α oderein TNF-R2:Ig-Fusionsprotein), einem Liganden von CCR5 (vorzugsweise RANTES (auch als "CCL5" bezeichnet) oder MIP-1α (auch als "CCL3" bezeichnet)) und einem 2- oder 3-wertigen Kation, bevorzugt in Form eines löslichen Salzes von Calcium, Aluminium, Gadolinium, Strontium oder Magnesium.

In einer bevorzugten Ausgestaltung der Erfindung erfolgt parallel zu der spezifischen Aktivierung eines Signaltransduktionsweges durch das stimulierende Agens ein sogenanntes unspezifisches Priming der Zellen über Toll-like Rezeptoren (TLR). Dazu enthält das Röhrchen mit dem stimulierenden Agens zusätzlich mindestens einen Toll-like-Rezeptor Liganden, bevorzugt ausgewählt aus LPS, abgetöteten Bakterien, ssRNA, dsRNA, CpG-Oligonukleotiden, Zymosan und Hitzeschockproteinen. Bevorzugt enthält das Röhrchen mit dem stimulierenden Agens für das unspezifische Priming mit dem TLR-Liganden eine nicht-aktivierende Konzentration eines TLR-Liganden. Als nicht-aktivierende Konzentration eines TLR-Liganden im Sinne der Erfindung ist eine Menge des TLR-Liganden zu verstehen, die für sich genommen nicht geeignet ist, um eine unspezifische Anregung von Signaltransduktionswegen in den Zellen zu ermöglichen. Besonders bevorzugt ist in dem Röhrchen mit dem stimulierenden Agens daher ein TLR-Ligand in einer Menge enthalten, so dass bei Befüllung des Röhrchens mit der Vollblutprobe eine Konzentration von maximal 1 ng/ml (die Konzentration bezieht sich auf die Menge des TLR-Liganden je Milliliter Vollblutprobe), besonders bevorzugt maximal 0,1 ng/ml, weiter besonders bevorzugt 0,01 bis 1 ng/ml, ganz besonders bevorzugt 0,01 bis 0,1 ng/ml erreicht wird. Bevorzugte TLR-Liganden, die für das unspezifische Priming eingesetzt werden, sind ausgewählt aus LPS, durch Hitze abgetötete *Listeria monocytogenes,* ssRNA40, Tenascin, oder Flaggelin. Besonders bevorzugt ist LPS. Durch das unspezifische Priming wird mit der nicht-aktivierenden Konzentration eines TLR-Liganden wird vorteilhaft die Reaktion der Zellen auf die spezifische Stimulation verbessert. Überraschend konnten im Vergleich zur ausschließlichen spezifischen Stimulation der Zellen mit dem stimulierenden Agens (ohne unspezifisches Priming) in dieser Ausgestaltung der Erfindung besonders reproduzierbare Ergebnisse durch den Zusatz des niedrigkonzentrierten TLR-Liganden erzielt werden.

Nach der Kultivierung wird der zellfreie Überstand der kultivierten Vollblutproben gewonnen, vorzugsweise mittels Zentrifugation. Aus dem Überstand wird dann die Konzentration mindestens zweier, besonders bevorzugt mindestens dreier, der Zytokine TNF-α, IL-8, IFNγ, IL-10, IL-1β und/oder IL-6 bestimmt. Ebenfalls möglich ist die Konzentrationsbestimmung von einem oder mehreren gelösten Rezeptoren, bevorzugt ausgewählt aus TNFR1, TNFR2, IL1R Antagonist, IL-1R1, IL-1R2. Die Bestimmung der Konzentration von Zytokinen oder gelösten Rezeptoren kann mit verschiedenen bekannten Methoden, wie ELISA oder auch cytrometric bead assays erfolgen. Vorzugsweise wird aufgrund der einfachen Handhabung ein ELISA zur Bestimmung der Zytokinkonzentration eingesetzt.

Mit der Erfindung wird durch die spezifische Aktivierung eines bestimmten Signaltransduktionsweges in Monozyten eines Patienten, die sich in einer Vollblutprobe befinden, ein Zytokinprofil erstellt, anhand dessen die prognostische Einschätzung des Krankheitsverlaufs und/oder die Diagnose der rheumatoiden Arthritis getroffen wird. Für die Erfindung besonders geeignet und damit bevorzugt sind die folgenden drei Analysen, die bereits jeweils einzeln die prognostische Einschätzung des Krankheitsverlaufs und/oder eine Diagnose der rheumatoiden Arthritis ermöglichen. Bevorzugt werden mindestens zwei der folgenden Analysen mit der Erfindung durchgeführt.

Erste Analyse: Vergleich der spontanen Sezernierung proinflammatorischer Zytokine mit der Sezernierung der proinflammatorischen Zytokine nach Quervernetzen des transmembranen TNF-α mit einem spezifischen Bindungspartner des transmembranen TNF-α (tmTNF-α). Bei diesem spezifischen Bindungspartner handelt es sich bevorzugt um anti-TNF-α oder ein TNF-R2:Ig-Fusionsprotein.

Für diese erste Analyse ist ein spezifischer Bindungspartner von tmTNF-α in einem Röhrchen als stimulierenden Agens enthalten. Bevorzugt sind 50 - 300 µg/ml, besonders bevorzugt 75 - 125 µg/ml des spezifischen Bindungspartners von tmTNF-α im Röhrchen enthalten. Dieses Röhrchen enthält vorzugsweise zusätzlich eine nicht-aktivierende Menge eines TLR-Liganden, insbesondere wie oben beschrieben.

Der spezifische Bindungspartner von tmTNF-α kann entweder oberflächengebunden oder gelöst vorliegen. Bevorzugt wird der spezifische Bindungspartner von tmTNF-α in oberflächengebundener Form eingesetzt. Liegt der spezifische Bindungspartner gebunden vor, wird vorzugsweise zusätzlich eine lösliche Form des spezifischen Bindungspartners mit einer Endkonzentration im Kulturmedium von 30 - 50 µg/ml, bevorzugt 40 µg/ml zugegeben. Liegt der spezifische Bindungspartner ausschließlich in gelöster Form vor, so beträgt seine Endkonzentration im Kulturmedium vorzugsweise 40 µg/ml.

Aus dem zellfreien Überstand der Vollblutproben aus den Röhrchen werden bei dieser ersten Analyse die Konzentrationen von mindestens zwei, vorzugsweise mindestens drei, der Zytokine TNF-α, IL-8, IFNγ, IL-10 und IL-1β, besonders bevorzugt aller der vorgenannten Zytokine, bestimmt. Optional werden zusätzlich die Konzentrationen von mindestens einem der Zytokine IL-6, IFNα, IL-1α, IL12, IL-23 und IL-4 bestimmt. Ebenfalls möglich ist die Konzentrationsbestimmung von einem oder mehreren gelösten Rezeptoren, bevorzugt ausgewählt aus TNFR1, TNFR2, IL1R Antagonist, IL-1R1, IL-1R2.

Diese erste Analyse beruht auf der Beobachtung, dass Monozyten von Patienten mit rheumatoider Arthritis bereits spontan proinflammatorische Zytokine, insbesondere IL-1β, IL-1α, TNF-α, TNFR1 und IL-8 sezernieren. Ein Quervernetzen des transmembranen TNF-α mit dem spezifischen Bindungspartner von tm-TNF-α hat inhibitorische Wirkung auf die Sezernierung von IL-1β. Bei dieser ersten Analyse zeigt damit sowohl eine erhöhte spontane IL-1β Produktion in der Negativkontrolle als auch eine geringere Konzentration von IL-1β im Überstand der Probe des Röhrchens mit dem spezifischen Bindungspartner von tm-TNF-α im Vergleich zur Negativkontrolle das Risiko für das Vorliegen einer rheumatoiden Arthritis an.

Bei den folgenden Zytokinen weist eine erhöhte Konzentration im Überstand der Probe des Röhrchens mit dem spezifischen Bindungspartner des transmembranen TNF-α im Vergleich zur Negativkontrolle auf ein erhöhtes Risiko für das Vorliegen von RA hin: TNF-α, IL-8, IFNγ, IL-6, INFα, IL-1α, IL-23.

Eine Zytokinkonzentration der Zytokine TNF-α und IL-8 im Überstand der Probe des Röhrchens mit dem spezifischen Bindungspartner des transmembranen TNF-α von bevorzugt 200 pg/ml, besonders bevorzugt 500 pg/ml, weist auf einen pathologischen Befund hin.

Bei den folgenden Zytokinen weist eine erniedrigte Konzentration im Überstand der Probe des Röhrchens mit dem spezifischen Bindungspartner des transmembranen TNF-α im Vergleich zur Negativkontrolle auf ein erhöhtes Risiko für das Vorliegen von RA hin: IL-10, IL-4, IL-12.

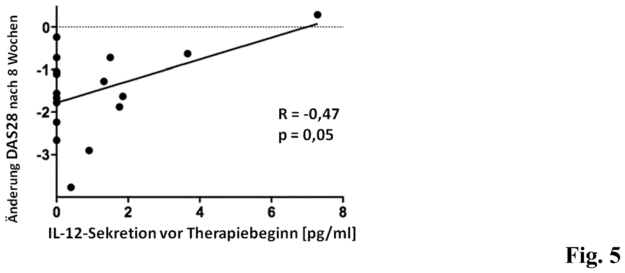

Beobachtungen der Erfinder haben gezeigt, dass die spontane Sezernierung von proinflammatorischen Zytokinen sowie von gelösten Zytokinrezeptoren (insbesondere von IL-1β, IL-1α, TNFR1) von Monozyten aus Patienten mit rheumatoider Arthritis, invers mit dem Ansprechen auf eine TNF-Inhibitor-Therapie, gemessen am Abfall des DAS28-Wertes, korreliert (vgl. Fig. 1, 5). Bei den folgenden Zytokinen weist eine geringere Konzentration im Überstand der Probe des Röhrchens mit dem spezifischen Bindungspartner des transmembranen TNF-α im Vergleich zur Negativkontrolle auf ein schlechtes Ansprechen auf eine TNF-Inhibitor-Therapie hin: IL-1β, IL-1α, TNFR1.

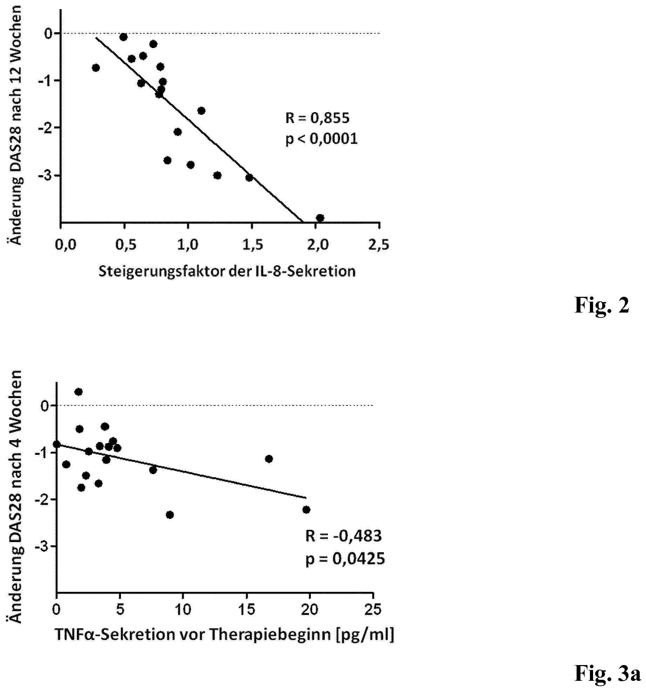

Beobachtungen der Erfinder haben weiter gezeigt, dass die Sezernierung von einigen proinflammatorischen Zytokinen sowie von gelösten Zytokinrezeptoren (insbesondere von IL-8, TNF-a, TNFR1, IL1R1) von Monozyten aus Patienten mit rheumatoider Arthritis nach Quervernetzung des transmembranen TNF-α mit dem spezifischen Bindungspartner von tm-TNF-α, mit dem Ansprechen auf eine TNF-Inhibitor-Therapie, gemessen am Anstieg des DAS28-Wertes, korreliert (vgl. Fig. 2, 3 und 4). Bei den folgenden Zytokinen weist eine erhöhte Konzentration im Überstand der Probe des Röhrchens mit dem spezifischen Bindungspartner des transmembranen TNF-α im Vergleich zur Negativkontrolle auf ein gutes Ansprechen auf eine TNF-Inhibitor-Therapie hin: IL-8, TNF-α, TNFR1, IL1R1; besonders bevorzugt von IL-8 (siehe Fig. 2).

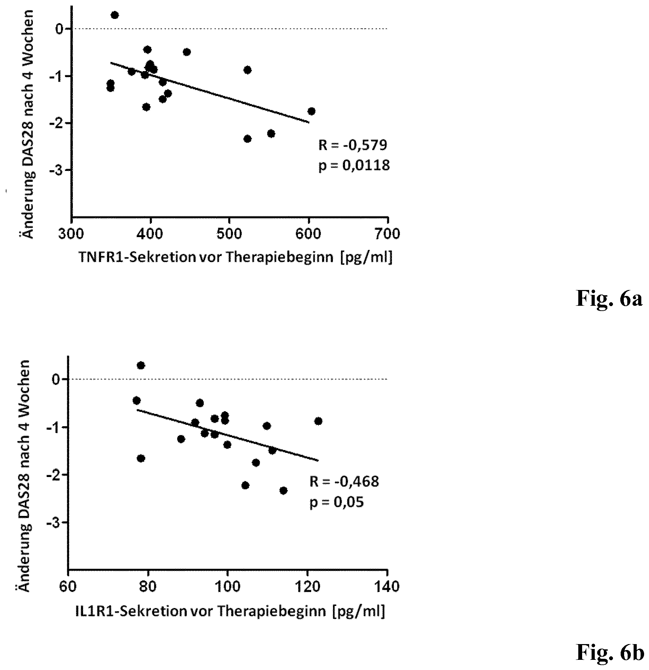

Beobachtungen der Erfinder haben weiter gezeigt, dass die Sezernierung von einigen proinflammatorischen Zytokinen sowie von gelösten Zytokinrezeptoren (insbesondere von IL-12, IL-1β) von Monozyten aus Patienten mit rheumatoider Arthritis nach Quervernetzung des transmembranen TNF-α mit dem spezifischen Bindungspartner von tm-TNF-α, invers mit dem Ansprechen auf eine TNF-Inhibitor-Therapie, gemessen am Anstieg des DAS28-Wertes, korreliert (vgl. Fig. 5, 6b). Bei den folgenden Zytokinen weist eine erhöhte Konzentration im Überstand der Probe des Röhrchens mit dem spezifischen Bindungspartner des transmembranen TNF-α im Vergleich zur Negativkontrolle auf ein schlechtes Ansprechen auf eine TNF-Inhibitor-Therapie hin: IL-12, IL-1β.

Zur Bestimmung der Konzentration von TNF-α wird besonders bevorzugt als spezifischer Bindungspartner von tmTNF-α ein TNF-R2:Ig-Fusionsprotein verwendet.

### Zweite Analyse: Auslösung des CCR5-Signalwegs

Für diese zweite Analyse ist ein Ligand von CCR5 (CC-Motiv-Chemokin-Rezeptor 5) in einem Röhrchen als stimulierenden Agens enthalten. Dieses Röhrchen enthält vorzugsweise zusätzlich eine nicht-aktivierende Menge eines TLR-Liganden, insbesondere wie oben beschrieben. Bevorzugt ist der Ligand von CCR5 ausgewählt aus RANTES (CCL5) oder MIP-1α (CCL3). Der Ligand von CCR5 liegt dabei im Röhrchen vorzugsweise löslich vor. RANTES wird in einer Konzentration von 0,1 - 10 µg/ml, bevorzugt 0,8 - 1,2 µg/ml, besonders bevorzugt 1 µg/ml und MIP-1a wird in einer Konzentration zwischen 0,1 nmol/l und 1 nmol/l eingesetzt. Aus dem zellfreien Überstand der Vollblutproben aus den Röhrchen werden bei dieser zweiten Analyse die Konzentrationen von mindestens einem, vorzugsweise mindestens zwei, der Zytokine TNF-α, IL-6 und IFNγ, besonders bevorzugt aller der vorgenannten Zytokine, bestimmt. Optional werden zusätzlich die Konzentrationen von mindestens einem der Zytokine IL-1α, IL-1β, IL-23 und IL-8 bestimmt.

Diese zweite Analyse beruht auf der Beobachtung, dass die Stimulation des Chemokinrezeptors CCR5 auf T-Zellen und Monozyten zur Sezernierung proinflammatorischer Zytokine, insbesondere TNF-α, IL-6 und IFNγ, führt, welche bei Trägern der genetischen Deletion (etwa 10 % der Europäer) vermindert ist. Bei dieser zweiten Analyse zeigt damit eine verminderte Konzentration von mindestens einem, vorzugsweise mindestens zwei, besonders bevorzugt allen der Zytokine TNF-α, IL-6, IL-8 und IFNγ im Überstand der Probe des Röhrchens mit dem Liganden von CCR5 im Vergleich zum Normwert, ermittelt aus den Proben von 5 gesunden Individuen ohne die genetische Deletion von CCR5, ein vermindertes Risiko für das Vorliegen von RA an. Auch das zusätzliche Vorliegen einer erniedrigten Konzentration von mindestens einem, vorzugsweise mindestens zwei, besonders bevorzugt mindestens drei der Zytokine IL-1α, IL-1β, IL-23 und IL-8 im Überstand der Probe des Röhrchens mit dem Liganden von CCR5 im Vergleich zum Normwert von Individuen ohne die genetische Deletion von CCR5 zeigt ein partiell erniedrigtes Risiko für das Vorliegen von RA an. Ebenfalls möglich ist die Konzentrationsbestimmung von einem oder mehreren gelösten Rezeptoren, bevorzugt ausgewählt aus TNFR1, TNFR2, IL1R Antagonist, IL-1R1, IL-1R2.

Die prognostische Einschätzung des Krankheitsverlaufs auf Basis dieser zweiten Analyse beruht auf der Beobachtung, dass RA-Patienten mit einer heterozygoten Deletion im CCR5-Gen einen milden Krankheitsverlauf und eine gute Prognose haben. Wird die zweite Analyse daher mit einer Vollblutprobe durchgeführt, die nachweislich von einem RA-Patienten stammt (vorzugsweise durch Diagnose mit Hilfe der ersten oder dritten Analyse), so zeigt das Vorliegen von einer erniedrigten Konzentration der nachgewiesenen Zytokine (vorzugsweise alle Zytokine TNF-α, IL-6 und IFNγ) im Überstand der Probe des Röhrchens mit dem Liganden von CCR5 im Vergleich zum Normwert von Individuen ohne die genetische Deletion von CCR5 eine gute Prognose für die RA-Erkrankung an. Vorzugsweise beträgt für die eine gute Prognose anzeigende Konzentration eines Zytokins im Röhrchen mit dem CCR5-Liganden maximal +/- 10 % der Konzentration der Negativkontrolle.

### Dritte Analyse: Auslösung des GPRC6A-Signalwegs

Für diese dritte Analyse ist ein 2- oder 3-wertiges Kation, bevorzugt in Form eines löslichen Salzes von Calcium, Aluminium, Gadolinium, Strontium oder Magnesium, in einem Röhrchen als stimulierenden Agens enthalten. Die 2- oder 3-wertigen Kationen liegen bevorzugt als lösliches Salz vor. Als Anionen sind besonders bevorzugt Chlorid, Acetat oder Citrat. Bevorzugt ist der Einsatz eines der folgenden Salze, wobei in der untenstehenden Tabelle 1 die jeweils bevorzugte Arbeitskonzentration angegeben ist. Es ist in dem Röhrchen mit dem stimulierenden Agens jene Menge des 2- oder 3-wertigen Kations enthalten, dass nach Zugabe des Volumens der Vollblutprobe zum Röhrchen die jeweilige Arbeitskonzentration erzielt wird.

**Tabelle 1**

| **Salz** | **bevorzugte Arbeitskonzentrationen** |
|---|---|
| Calciumchlorid | 1,5 - 2,5 mmol/l |
| Aluminiumchlorid (AlCl₃x6H₂O) | 300 - 1000 µmol/l |
| Gadoliniumchlorid- Hexahydrat | 300 - 1000 µmol/l |
| Magnesiumchlorid | 30 - 50 mmol/l |
| Strontiumchlorid | 0,2 - 0,5 mmol/l |

Das 2- oder 3-wertigen Kation liegt dabei im Röhrchen gelöst vor.

Bevorzugt enthält das Röhrchen mit dem 2- oder 3-wertigen Kation vorzugsweise zusätzlich TLR-Liganden, um das Ansprechend der Zellen auf die spezifische Stimulation zu verbessern. Das Röhrchen mit dem 2- oder 3-wertigen Kation enthält dabei vorzugsweise zusätzlich eine nicht-aktivierende Menge eines TLR-Liganden, insbesondere wie oben beschrieben. Bevorzugt ist alternativ dazu in dem Röhrchen mit dem 2- oder 3-wertigen Kation neben den stimulierenden Agens eine aktivierende Menge eines TLR-Liganden, insbesondere ausgewählt aus LPS, durch Hitze abgetöteten *Listeria monocytogenes*, Poly(I:C), Flagellin und ssRNA40, enthalten. LPS ist dabei vorzugsweise in einer Konzentration von 50 - 150 ng/ml, besonders bevorzugt von 100 ng/ml enthalten. Durch Hitze abgetötete *Listeria monocytogenes* sind bevorzugt in einer Konzentration von 10⁷ bis 10⁹, besonders bevorzugt von maximal 10⁸ Zellen pro ml, enthalten. Poly(I:C) ist bevorzugt in einer Konzentration von 8 bis 12 µg/ml, besonders bevorzugt von maximal 10 µg/ml enthalten. Flagellin ist bevorzugt in einer Konzentration von 0,8 bis 1,2 µg/ml, besonders bevorzugt von maximal 1 µg/ml enthalten. SsRNA40 ist bevorzugt in einer Konzentration von 0,8 bis 1,2 µg/ml, besonders bevorzugt von maximal 1 µg/ml. Im Rahmen der dritten Analyse werden reproduzierbare Ergebnisse insbesondere durch Co-Stimulation mit einem TLR-Liganden in aktivierender Menge erzielt.

Aus dem zellfreien Überstand der Vollblutproben aus den Röhrchen wird bei dieser dritten Analyse die Konzentration von IL-1β bestimmt. Optional werden zusätzlich die Konzentrationen von IL-6, IL-23 und/oder TNF-α bestimmt. Ebenfalls möglich ist die Konzentrationsbestimmung von einem oder mehreren gelösten Rezeptoren, bevorzugt ausgewählt aus TNFR1, TNFR2, IL1R Antagonist, IL-1R1, IL-1R2.

Diese dritte Analyse beruht auf der Beobachtung, dass die Stimulation des G-Protein gekoppelten Rezeptors GPRC6A in Monozyten bei Vorliegen einer rheumatoiden Arthritis die Sezernierung von IL-1β, und weiterhin auch von IL-6, IL-23 und/oder TNF-α, vermittelt. In der dritten Analyse zeigt daher eine höhere Konzentration von IL-1 β im Überstand der Probe des Röhrchens mit dem 2- oder 3-wertigen Kation im Vergleich zur Negativkontrolle ein erhöhtes Risiko für das Vorliegen von RA an. Das zusätzliche Vorliegen einer höheren Konzentration von IL-6, IL-23 und/oder TNF-α im Überstand der Probe des Röhrchens dem 2- oder 3-wertigen Kation im Vergleich zur Negativkontrolle zeigt ein erhöhtes Risiko für das Vorliegen von RA an. Ebenfalls möglich ist die Konzentrationsbestimmung von einem oder mehreren gelösten Rezeptoren, bevorzugt ausgewählt aus TNFR1, TNFR2, IL1R A01223R0001DEntagonist, IL-1R1, IL-1R2. In der Negativkontrolle sind keine löslichen Rezeptoren nachweisbar. Erhöhte Spiegel der Rezeptoren deuten jeweils auf erhöhtes Risiko hin.

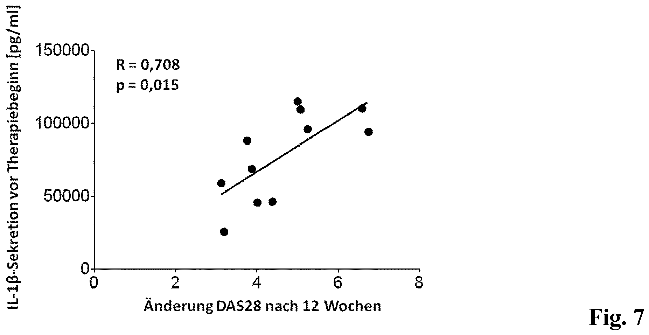

Beobachtungen der Erfinder haben demonstriert, dass die Stimulation von GPRC6A mittels erhöhter extrazellulärer Kationen-Konzentrationen als prognostischer Parameter zur Prognose des zu erwartenden Ansprechens auf die Therapie mit einem TNF-Inhibitor geeignet ist (vgl. Fig. 7). In der dritten Analyse zeigt daher eine höhere Konzentration von IL-1β im Überstand der Probe des Röhrchens mit dem 2- oder 3-wertigen Kation in Kombination mit einer aktivierenden Menge eines TLR-Liganden im Vergleich zur Kontrolle mit einer aktivierenden Menge eines TLR-Liganden (jedoch ohne stimulierendes Agens) eine gute Prognose hinsichtlich des Ansprechens auf eine Therapie mit einem TNF-Inhibitor an.

Bevorzugt wird bei der Erfindung mindestens eine der drei vorgenannten Analysen durchgeführt. Besonders bevorzugt wird mindestens die erste Analyse durchgeführt. Ganz besonders bevorzugt werden die erste und die zweite Analyse in Kombination miteinander durchgeführt. Dies erlaubt vorteilhaft eine diagnostische und eine prognostische Aussage hinsichtlich der Erkrankung. Zur besseren Diagnose- und Prognosesicherung werden mit der Erfindung alle drei Analysen durchgeführt. Werden mehrere Analysen (erste, zweite, dritte) vorgenommen, so findet die Stimulation mit den jeweils unterschiedlichen stimulierenden Agentien in separaten Röhrchen statt.

Von der Erfindung ist auch die Verwendung eines diagnostischen Kits zur Diagnose der rheumatoiden Arthritis oder prognostischen Einschätzung des Krankheitsverlaufs der rheumatoiden Arthritis umfasst, mit dem zumindest Teilschritte mindestens einer der drei vorgenannten Analysen (erste, zweite, dritte Analyse), durchführbar sind. Das erfindungsgemäß verwendete diagnostische Kit umfasst:
- mindestens ein Röhrchen, enthaltend:
   ∘ ein stimulierendes Agens, ausgewählt aus einem spezifischen Bindungspartner von tmTNF-α, vorzugsweise anti-TNF-α oder TNFR2:Ig (bevorzugt in löslicher oder oberflächengebundener Form, besonders bevorzugt zumindest in oberflächengebundener Form), einem CCR5- Ligand, vorzugsweise RANTES oder MIP-1α, und/oder einem 2-oder 3-wertigen Kation, bevorzugt der Metalle Calcium, Aluminium, Gadolinium, Strontium oder Magnesium, bevorzugt in Form eines löslichen Salzes,
   ∘ mindestens einen TLR-Liganden, vorzugsweise in einer nicht-aktivierenden Menge, wie oben beschrieben,
- mindestens zwei Vergleichsröhrchen, von denen ein erstes Röhrchen ein unbehandeltes Leerröhrchen für die Negativkontrolle ist und ein zweites Röhrchen enthaltend Lipopolysaccharid für die Positivkontrolle,
- ggf. Mittel zur Bestimmung der Zytokinkonzentration, vorzugsweise mindestens eine ELISA-Platte zur Bestimmung der Konzentration mindestens eines, bevorzugt mindestens zwei, besonders bevorzugt mindestens drei der Zytokine α, IL-8, IFNγ, IL-10, IL-1β und IL-6.
- ggf. Mittel zur Bestimmung der Konzentration gelöster Rezeptoren, ausgewählt aus TNFR1, TNFR2, IL1R Antagonist, IL-1R1, IL-1R2.

Bevorzugt sind in dem erfindungsgemäß verwendeten diagnostischen Kit mehrere Röhrchen mit unterschiedlichen der genannten stimulierenden Agenzien enthalten.

Ein erfindungsgemäß verwendetes diagnostisches Kit enthält damit die notwendigen Mittel, um Vollblutproben eines menschlichen Individuums, bei dem der Verdacht einer Erkrankung mit rheumatoider Arthritis besteht, mit einen stimulierenden Agens zu versetzen. Die Proben können dann direkt in dem Röhrchen bei der Temperatur von 30 - 40 °C inkubiert werden. Die Analyse der während der Inkubation mit dem stimulierenden Agens gebildeten Zytokine kann mit Mitteln, die nicht zwingend Bestandteil des diagnostischen Kits sind durchgeführt werden. Bevorzugt sind auch die Mittel für die Zytokinanalyse im erfindungsgemäß verwendeten diagnostischen Kit enthalten.

Bevorzugt ist mindestens ein Röhrchen mit anti-TNF-α als stimulierendem Agens im diagnostischen Kit enthalten. Dieses enthält bevorzugt eine nicht-aktivierende Menge eines TLR-Liganden, wie oben beschrieben. Vorzugsweise enthält das Röhrchen maximal 1 ng/ml des TLR-Liganden, insbesondere LPS. Bevorzugt ist mindestens ein Röhrchen mit einem Liganden von CCL5 als stimulierendem Agens, vorzugsweise mit RANTES oder MIP-1α, im diagnostischen Kit enthalten. Dieses enthält bevorzugt eine nicht-aktivierende Menge eines TLR-Liganden, wie oben beschrieben. Vorzugsweise enthält das Röhrchen maximal 1 ng/ml des TLR-Liganden, insbesondere LPS. Bevorzugt ist mindestens ein Röhrchen mindestens ein Röhrchen mit einem 2- oder 3-wertigen Kation bevorzugt in Form eines löslichen Salzes von Calcium, Aluminium, Gadolinium, Strontium oder Magnesium als stimulierendem Agens im diagnostischen Kit enthalten. Dieses enthält bevorzugt einen TLR-Liganden, insbesondere LPS.

Bevorzugt ist mindestens ein Röhrchen mit anti-TNF-α als stimulierendem Agens und mindestens ein Röhrchen mit einem Liganden von CCL5, vorzugsweise mit RANTES oder MIP-1α, im diagnostischen Kit enthalten.

Besonders bevorzugt ist mindestens ein Röhrchen mit anti-TNF-α als stimulierendem Agens, mindestens ein Röhrchen mit einem Liganden von CCL5, vorzugsweise mit RANTES oder MIP-1α, und mindestens ein Röhrchen mit einem 2- oder 3-wertigen Kation bevorzugt in Form eines löslichen Salzes von Calcium, Aluminium, Gadolinium, Strontium oder Magnesium, im diagnostischen Kit enthalten.

Die Ausgestaltung der Röhrchen im diagnostischen Kit erfolgt bevorzugt wie oben beschrieben. Bevorzugt sind die Röhrchen für den Einsatz in einem erfindungsgemäß verwendeten diagnostischen Kit als Blutentnahmeröhrchen ausgestaltet, vorzugsweise Blutentnahmeröhrchen nach dem Unterdrucksystem. Die Röhrchen sind vorzugsweise auf der Innenseite mit einem bioaktiven Protein beschichtet.

Das Röhrchen für die Positivkontrolle enthält bevorzugt 200 - 900 ng Lipopolysaccharid.

Zur Bestimmung der Zytokinkonzentration in dem aus der inkubierten Vollblutprobe gewonnenen zellfreien Überstand werden bevorzugt ELISA-Verfahren (enzyme linked immunosorbent assay) eingesetzt. Dafür ist für den Nachweis eines Zytokins im erfindungsgemäßen diagnostischen Kit bevorzugt Material zumNachweis des jeweiligen Zytokins enthalten, besonders bevorzugt in Form einer jeweiligen ELISA-Platte. Dafür ist die ELISA-Platte (eine 96-well Platte) bevorzugt mit einem Antikörper beschichtet, der das jeweilig nachzuweisende Zytokin spezifisch bindet. Bevorzugt ist daher mindestens eine ELISA-Platte im diagnostischen Kit enthalten die mit einem spezifischen Antikörper gegen ein Zytokin, ausgewählt aus TNF-α, IL-8, IFNγ, IL-10, IL-1β oder IL-6, oder einen gelösten Rezeptor, ausgewählt aus TNFR1, TNFR2, IL1R-Antagonist, IL-1R1, IL-1R2 beschichtet ist.

Bevorzugt umfasst ein erfindungsgemäß verwendetes diagnostisches Kit:
- für die erste Analyse: mindestens ein Röhrchen mit anti-TNF-a und Material zum Nachweis der Konzentration eines der Zytokine TNF-α, IL-8, IFNγ, IL-10 und IL-1β und/oder einem der gelösten Rezeptoren TNFR1, TNFR2, IL1R-Antagonist, IL-1R1, IL-1R2 (bevorzugt ist das Material zum Nachweis der Zytokinkonzentration bzw. der Konzentration der gelösten Rezeptoren mindestens eine ELISA-Platte, besonders bevorzugt sind mindestens zwei, weiter besonders bevorzugt mindestens drei, ganz besonders bevorzugt mindestens vier ELISA-Platten enthalten, die zum Nachweis der Konzentration jeweils eines der vorgenannten Zytokine und/oder gelösten Rezeptoren vorgesehen ist),
- für die zweite Analyse: mindestens ein Röhrchen mit einem Liganden von CCR5, vorzugsweise RANTES oder MIP-1α, und Material zum Nachweis der Konzentration eines der Zytokine TNF-α, IL-6 und IFNγ und/oder einem der gelösten Rezeptoren TNFR1, TNFR2, IL1R Antagonist, IL-1R1, IL-1R2 vorgesehen ist ((bevorzugt ist das Material zum Nachweis der Zytokinkonzentration bzw. der Konzentration der gelösten Rezeptoren mindestens eine ELISA-Platte, besonders bevorzugt sind mindestens zwei, weiter besonders bevorzugt mindestens drei ELISA-Platten enthalten, die zum Nachweis der Konzentration jeweils eines der vorgenannten Zytokine und/oder gelösten Rezeptoren vorgesehen ist), und/oder
- für die dritte Analyse: mindestens ein Röhrchen mit einem 2- oder 3-wertigen Kation bevorzugt in Form eines löslichen Salzes von Calcium, Aluminium, Gadolinium, Strontium oder Magnesium und Material zum Nachweis von IL-1β, IL-6, IL-23, TNF-α, TNFR1, TNFR2, IL1R Antagonist, IL-1R1 und/oder IL-1R2 bevorzugt in Form einer ELISA-Platte.

Optional sind im erfindungsgemäß verwendeten diagnostischen Kit für die jeweilige Analyse weitere Materialien, vorzugsweise ELISA-Platten enthalten, die zum Nachweis der o.g. optional analysierten Zytokine vorgesehen sind.

Neben der oder den ELISA-Platten sind bevorzugt die folgenden weiteren Mittel zur Bestimmung der Zytokinkonzentration mittels ELISA im diagnostischen Kit enthalten:
- ein weiter Antikörper, der mit dem Antikörper auf der ELISA-Platte nicht identisch ist, und der das jeweilig nachzuweisende Zytokin spezifisch bindet. Dieser weitere Antikörper ist bevorzugt mit einem Reporterenzym konjugiert (vorzugsweise Meerettichperoxidase, Alkalische Phosphatase oder Glucoseoxidase).
- ein Farbstoffsubstrat (vorzugsweise p-Nitrophenylphosphat oder o-Phenylendiamin), und
- ein Standard, enthaltend das jeweilig nachzuweisende Zytokin (in bekannter Konzentration).

Die Erfindung umfasst auch die Verwendung eines beschriebenen Kits zur prognostischen Einschätzung zur Schwere einer Gelenksdestruktion im Rahmen der rheumatoiden Arthritis. Besonders bevorzugt erfolgt mit der Erfindung eine prognostische Einschätzung des Krankheitsverlaufs, insbesondere die prognostische Einschätzung des Krankheitsverlaufs von rheumatoider Arthritis bei Therapie mit einem TNF-blockierenden Arzneimittel. Bevorzugt erfolgt diese Verwendung in einem erfindungsgemäßen Verfahren.

Die Erfindung gibt ein Verfahren und ein diagnostisches Kit an, mit dem es möglich ist, in einem einfachen, kostengünstigen "in tube"-Test eine prognostische Aussage zum Krankheitsverlauf der RA und ggf. zusätzlich auch eine frühzeitige Diagnose von RA zu stellen. Weiterhin ist es möglich, im Rahmen der prognostischen Einschätzung eines Aussage hinsichtlich des Ansprechens des Patienten auf eine bestimmte Therapie (die sogenannte "Therapieresponse", insbesondere eine Prognose der Therapieresponse auf eine Therapie mit einem TNF-inhibierenden Arzneimittel) zu treffen.

Der Nachweis der Zytokinproduktion von Zellen des peripheren Blutes erlaubt eine belastbarere Aussage über den Krankheitsstatus und die Krankheitsprognose als die alleinige Analyse von Autoantikörpern, die je nach Inflammationsstatus auch stets entsprechende Immunzellen im peripheren Blut vorliegen. Es wird damit bei der Erfindung erstmalig der der Aktivierungsstatus der an der Erkrankung beteiligten Immunzellen berücksichtigt.

Anhand folgender Figuren und Ausführungsbeispiele soll die Erfindung näher erläutert werden, ohne dabei auf diese beschränkt zu werden.
**Fig. 1a****-c** zeigen, dass die spontane monozytäre in vitro-Zytokinproduktion, sowie die spontane monozytäre Sekretion von gelösten Zytokinrezeptoren vor Therapiebeginn invers mit dem Ansprechen auf eine TNF-Inhibitor-Therapie, gemessen am Abfall des DAS28-Wertes, korreliert. Der DAS28-Wert (disease activity score) ist ein Wert zur Beurteilung der Krankheitsaktivität von rheumatoider Arthritis, in den die Anzahl der druckschmerzhaften Gelenke (0-28 Gelenke), die Anzahl der geschwollenen Gelenke (0-28), die Blutkörperchensenkungsgeschwindigkeit (mm/h) sowie die Einschätzungen des Krankheitszustandes durch den Patienten mit einfließen. Je höher dabei der DAS28-Wert, umso höher ist auch die Krankheitsaktivität. Fig. 1a bezieht sich dabei auf die spontane IL-1α-Sekretion, Fig. 1b auf die spontane IL-1β-Sekretion und Fig. 1c auf die spontane Sekretion des gelösten Zytokinrezeptors TNFR1.
   Eine erhöhte spontane monozytäre in vitro-Zytokinproduktion, sowie eine spontane monozytäre Sekretion von gelösten Zytokinrezeptoren ist ein diagnostischer Parameter, der mit RA assoziiert ist.
**Fig. 2** zeigt, dass die durch lösliches TNFR2:Ig induzierte Steigerung der spontanen monozytären IL-8-Sekretion *in vitro* mit dem Abfall der Erkrankung unter Therapie mit dem TNF Inhibitor, gemessen am Abfall des DAS28-Wertes, korreliert. Je höher dabei der Steigerungsfaktor der IL-8-Sekretion, umso stärker ist das Absenken des DAS28-Wertes nach 12 Wochen Therapie, umso besser demnach das Ansprechen auf die Therapie.

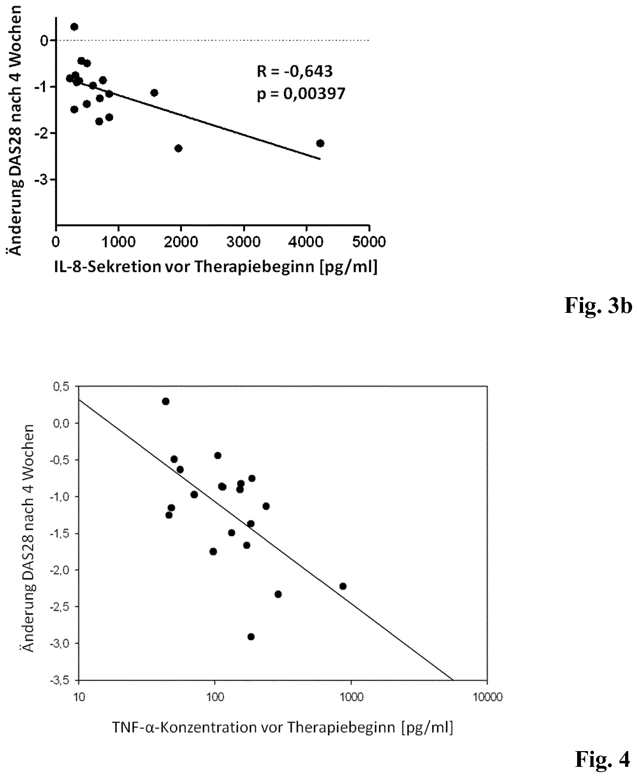

**Fig. 3a** **und** **3b** zeigen, dass die Höhe der durch Inkubation mit löslichem TNFR2:Ig induzierten monozytären Zytokinproduktion *in vitro* vor Therapie positiv mit dem Ansprechen auf eine TNF-Inhibitor-Therapie nach 4 Wochen, gemessen am Abfall des DAS28-Wertes, korreliert. Dabei bezieht sich Fig. 3a auf die TNFR2:Ig induzierte TNF-α-Sekretion und Fig. 3b auf die TNFR2:Ig induzierte IL-8-Sekretion vor Therapiebeginn.
**Fig. 4** zeigt, dass die durch tmTNF crosslinking und reverse signalling ausgelöste TNFα-Produktion eine signifikante Korrelation mit dem Ansprechen auf therapeutische TNF-Inhibition, gemessen am Abfall des DAS28-Wertes nach 4 Wochen Therapie, zeigt. Die Ergebnisse belegen, dass eine starke TNFa-Response auf tmTNF reverse signalling, induziert durch gebundenes TNFR2:Ig, ein Prädiktor für ein sehr gutes Ansprechen auf eine Therapie mittels TNFα-Inhibitoren ist.
**Fig. 5** zeigt, dass die Höhe der durch Inkubation mit gebundenem TNFR2:Ig induzierten monozytären IL-12-Sekretion *in vitro* vor Therapie negativ mit dem Ansprechen auf eine TNF-Inhibitor-Therapie nach 8 Wochen korreliert.
**Fig. 6a und 6b** zeigen, dass die Höhe der durch Inkubation mit gebundenem TNFR2:Ig induzierten monozytären Sekretion löslicher Zytokinrezeptoren in vitro vor Therapie positiv mit dem Ansprechen auf eine TNF-Inhibitor-Therapie nach 4 Wochen korreliert. Dabei bezieht sich Fig. 6a auf die TNFR2:Ig induzierte TNFR1-Sekretion und Fig. 6b auf die TNFR2:Ig induzierte IL1R1-Sekretion vor Therapiebeginn.
**Fig. 7** zeigt, dass die Stimulation von GPRC6A mittels erhöhter extra-zellulärer Calcium-Konzentration als prognostischer Parameter zur Prognose des zu erwartenden Ansprechens auf die Therapie mit einem TNF-Inhibitor geeignet ist. Die Ca²⁺-induzierte IL-1β-Produktion korreliert mit dem Abfall des DAS28 nach 12 Wochen Therapie, d.h. mit dem Ansprechen auf die Therapie.

### Beispiel 1: Analyse der TNF-α-Konzentration nach Inkubation mit gebundenem TNFR2:Ig

5 ml Stammlösung enthaltend 100 µg/ml TNFR2:Ig als stimulierendes Agens werden gegeben und für 3h bei 37 °C inkubiert. Der Überstand wird anschließend verworfen. Es wird erwartet, dass 125 µg TNFR2:Ig an der Oberfläche binden. Anschließend wird zusätzlich eine lösliche Form des TNFR2:Ig mit einer Endkonzentration im Kulturmedium von 40 µg/ml zugegeben.

12 ml Vollblut werden direkt nach der Entnahme mit Heparin, sowie mit 0,1 ng/ml LPS versetzt.

Jeweils 4 ml Vollblut werden anschließend in das Röhrchen, enthaltend gebundenes TNFR2:Ig, in ein Leerröhrchen als Negativkontrolle und in ein Röhrchen enthaltend Lipopolysaccharid als Positivkontrolle überführt.

Es folgt eine Inkubation für 16 h bei 37 °C. Anschließend werden die Proben für 30 Minuten bei 2000 rpm zentrifugiert und aus dem Zellfreien Überstand mittels ELISA die Konzentration von TNF-α bestimmt. Eine erhöhte Konzentration im Überstand der Probe des Röhrchens mit TNFR2:Ig im Vergleich zur Negativkontrolle weist auf ein erhöhtes Risiko für das Vorliegen von RA hin. Eine Korrelation mit dem Ansprechen auf therapeutische TNF-Inhibition, gemessen am Abfall des DAS28-Wertes nach 4 Wochen Therapie, ist in Fig. 4 gezeigt.

Tabelle 2 zeigt TNF-α-Konzentrationen von fünf gesunden Personen und 5 Personen mit Rheumatoider Arthritis, jeweils in dem Röhrchen für die Negativkontrolle und dem Röhrchen mit dem stimulierenden Agens.

**Tabelle 2**

| | | TNF-α-Konzentration pg/ml |
|---|---|---|
| Gesunde Kontrolle 1 | Negativkontrolle | 1 |
| | Geb. TNFR2:Ig | 701 |
| Gesunde Kontrolle 2 | Negativkontrolle | 0 |
| | Geb. TNFR2:Ig | 363 |
| Gesunde Kontrolle 3 | Negativkontrolle | 14 |
| | Geb. TNFR2:Ig | 157 |
| Gesunde Kontrolle 4 | Negativkontrolle | 4 |
| | Geb. TNFR2:Ig | 248 |
| Gesunde Kontrolle 5 | Negativkontrolle | 29 |
| | Geb. TNFR2:Ig | 404 |
| | | |
| RA-Patient 1 | Negativkontrolle | 31 |
| | Geb. TNFR2:Ig | 422 |
| RA-Patient 2 | Negativkontrolle | 49 |
| | Geb. TNFR2:Ig | 309 |
| RA-Patient 3 | Negativkontrolle | 19 |
| | Geb. TNFR2:Ig | 717 |
| RA-Patient 4 | Negativkontrolle | 19 |
| | Geb. TNFR2:Ig | 901 |
| RA-Patient 5 | Negativkontrolle | 3 |
| | Geb. TNFR2:Ig | 1298 |

### Beispiel 2: Auslösung des GPRC6A-Signalwegs

12 ml Vollblut werden direkt nach der Entnahme mit Heparin, sowie mit 0,1 ng/ml LPS versetzt.

In einem Röhrchen wird 2,5mM Calciumchlorid mit 100 ng/ml LPS versetzt.

4 ml Vollblut werden anschließend in das Röhrchen enthaltend 2,5mM Calciumchlorid gegeben, wodurch eine Endkonzentration von 1,7 mM Calciumchlorid erreicht wird. Jeweils weitere 4 ml werden in ein Leerröhrchen als Negativkontrolle und in ein Röhrchen enthaltend Lipopolysaccharid als Positivkontrolle überführt.

Es folgt eine Inkubation für 16 h bei 37 °C. Anschließend werden die Proben zentrifugiert und aus dem Zellfreien Überstand mittels ELISA die Konzentration von IL-1β bestimmt. In Fig. 7 ist gezeigt, dass die Ca²⁺-induzierte IL-1β-Produktion mit dem Abfall des DAS28 nach 12 Wochen Therapie korreliert.

### Zitierte Nichtpatentliteratur

Schellekens, G A, B A de Jong, F H van den Hoogen, L B van de Putte, and W J van Venrooij. "Citrulline Is An Essential Constituent of Antigenic Determinants Recognized by Rheumatoid Arthritis-specific Autoantibodies." The Journal of clinical investigation 101, no. 1 (1998): doi:10.1172/JCI1316.

Poulsom, Hannah, and Peter J Charles. "Antibodies to Citrullinated Vimentin Are a Specific and Sensitive Marker for the Diagnosis of Rheumatoid Arthritis." Clinical reviews in allergy & immunology 34, no. 1 (2008): doi:10.1007/sl2016-007-8016-3.

Meusch, Undine, Manuela Rossol, Christoph Baerwald, Sunna Hauschildt, and Ulf Wagner. "Outsideto-inside Signaling Through Transmembrane Tumor Necrosis Factor Reverses Pathologic Interleukin-1beta Production and Deficient Apoptosis of Rheumatoid Arthritis Monocytes." Arthritis and rheumatism 60, no. 9 (2009): doi:10.1002/art.24778.

Alex P, Szodoray P, Knowlton N, Dozmorov IM, Turner M, Frank MB, Arthur RE, Willis L, Flinn D, Hynd RF, Carson C, Kumar A, El-Gabalawy HS, Centola M. "Multiplex serum cytokine monitoring as a prognostic tool in rheumatoid arthritis." Clin Exp Rheumatol. 25 no. 4(2007): 584-92.

Holmgren C, Esplin MS, Hamblin S, Molenda M, Simonsen S, R. Silver R. "Evaluation of the use of anti-TNF-alpha in an LPS-induced murine model", Journal of Reproductive Immunology. 78, no. 2, 134-139 (2008) doi:10.1016/J.JRI.2007.11.003.

## Patentansprüche

1. Verfahren zur prognostischen Einschätzung des Krankheitsverlaufs bei rheumatoider Arthritis und/oder Diagnose der rheumatoiden Arthritis durch Zytokinanalyse aus einer menschlichen Vollblutprobe, wobei
- ein Volumen einer isolierten Vollblutprobe eines Menschen in mindestens ein Röhrchen mit einem stimulierenden Agens, überführt wird, wobei das stimulierenden Agens ein spezifischer Bindungspartner von tmTNF-a, ein Ligand von CCR5 oder ein 2- oder 3-wertiges Kation ist, und als Vergleichsproben das gleiche Volumen der Vollblutprobe in jeweils ein Leerröhrchen als Negativkontrolle und ein Röhrchen enthaltend Lipopolysaccharid als Positivkontrolle überführt wird,
- die jeweiligen Röhrchen mit den darin enthaltenen Vollblutproben anschließend bei einer Temperatur von 30 - 40 °C inkubiert werden,
- aus den Röhrchen der zellfreie Überstand der jeweiligen Vollblutprobe gewonnen wird und die Konzentration mindestens zwei Zytokinen ausgewählt aus TNF-α, IL-8, IFNγ, IL-10, IL-1β und IL-6 im zellfreien Überstand jeder Probe ermittelt wird,
- anhand einer veränderten Konzentration des mindestens einen Zytokins in dem mindestens einen Röhrchen mit dem stimulierenden Agens im Vergleich zur Negativkontrolle die prognostische Einschätzung des Krankheitsverlaufs der rheumatoiden Arthritis und/oder Diagnose der rheumatoiden Arthritis getroffen wird,
- wobei eine erhöhte Konzentration von TNF-α, IL-8, IFNγ, und IL-6 in der Probe mit dem spezifischen Bindungspartner von tmTNF-a im Vergleich zur Negativkontrolle das erhöhte Risiko für ein Vorliegen einer rheumatoiden Arthritis anzeigt,
wobei eine erniedrigte Konzentration von IL-1β oder IL-10 in der Probe mit dem spezifischen Bindungspartner von tmTNF-α im Vergleich zur Negativkontrolle das Vorliegen einer rheumatoiden Arthritis anzeigt,
wobei bei einer TNF-Inhibitor-Therapie eine erniedrigte Konzentration von IL-1β in der Probe mit dem spezifischen Bindungspartner von tmTNF-α im Vergleich zur Negativkontrolle auf ein schlechtes Ansprechen auf die Therapie und eine erhöhte Konzentration von TNF-α oder IL-8 in der Probe mit dem spezifischen Bindungspartner von tmTNF-α im Vergleich zur Negativkontrolle auf ein gutes Ansprechen auf die Therapie hinweist,
wobei eine erniedrigte Konzentration von TNF-α, IL-6, IL-8 und IFNγ in der Probe mit dem Liganden von CCR5 im Vergleich zum Normwert von Individuen ohne die genetische Deletion von CCR5 ein vermindertes Risiko für das Vorliegen einer rheumatoiden Arthritis oder in Patienten mit nachgewiesener rheumatoiden Arthritis eine gute Prognose für deren Verlauf anzeigt,
wobei eine erhöhte Konzentration von IL-1β in der Probe mit dem 2- oder 3-wertigen Kation im Vergleich zur Negativkontrolle ein erhöhtes Risiko für das Vorliegen einer rheumatoiden Arthritis anzeigt.

2. Verfahren nach einem der vorherigen Ansprüche, wobei die Vollblutprobe in dem mindestens einen Röhrchen mit dem stimulierenden Agens zusätzlich mit einer nicht aktivierenden Konzentration eines Toll-like-Rezeptor-Liganden versetzt wird.

3. Verfahren nach einem der vorherigen Ansprüche, wobei der spezifische Bindungspartner von tmTNF-α anti-TNF-α oder TNFR2:Ig ist.

4. Verfahren nach einem der vorherigen Ansprüche, wobei der CCR5 Ligand RANTES oder MIP-1α ist.

5. Verwendung eines Diagnostisches Kit, umfassend
- mindestens ein Röhrchen enthaltend
∘ mindestens ein stimulierendes Agens, ausgewählt aus einem spezifischen Bindungspartner von tmTNF-α, vorzugsweise anti-TNF-α oder TNFR2:Ig, einem CCR5- Ligand, vorzugsweise RANTES oder MIP-1α, und/oder einem 2- oder 3-wertigen Kation, bevorzugt der Metalle Calcium, Aluminium, Gadolinium, Strontium oder Magnesium, bevorzugt in Form eines löslichen Salzes,
∘ mindestens einen Toll-like-Rezeptor-Liganden,
- mindestens zwei Vergleichsröhrchen, von denen ein erstes Röhrchen ein unbehandeltes Leerröhrchen für die Negativkontrolle ist und ein zweites Röhrchen für die Positivkontrolle bakterielles Lipopolysaccharid enthält,
- ggf. Mittel zur Bestimmung der Zytokinkonzentration,
zur Diagnose oder prognostischen Einschätzung des Krankheitsverlaufs von rheumatoider Arthritis

6. Verwendung nach Anspruch 5, wobei das Röhrchen mit dem stimulierenden Agens eine nicht-aktivierende Menge eines Toll-like Rezeptor Liganden enthält.

7. Verwendung nach einem der Ansprüche 5 oder 6, umfassend mindestens ein Röhrchen mit einem spezifischen Bindungspartner von tmTNF-α, vorzugsweise anti-TNF-α oder TNFR2:Ig und/oder mindestens ein Röhrchen mit einem CCR5-Liganden, vorzugsweise RANTES oder MIP-1α und/oder mindestens ein Röhrchen mit einem 2- oder 3-wertigen Kation bevorzugt in Form eines löslichen Salzes von Calcium, Aluminium, Gadolinium, Strontium oder Magnesium .

8. Verwendung nach einem der Ansprüche 5 bis 7, wobei das Röhrchen mit dem stimulierenden Agens einen spezifischen Bindungspartner von tmTNF-α, vorzugsweise anti-TNF-α oder TNFR2:Ig in oberflächengebundener Form enthält.

9. Verwendung nach einem der Ansprüche 5 bis 8, umfassend als Röhrchen mit einem stimulierenden Agens
- mindestens ein Röhrchen mit einer nicht aktivierenden Menge eines Toll-like-Rezeptor-Liganden und einem spezifischen Bindungspartner von tmTNF-α, vorzugsweise anti-TNF- α oder TNFR2:Ig, und Material zum Nachweis der Konzentration eines der Zytokine TNF-α, IL-8, IFNγ, IL-10 und IL-1β ,
- mindestens ein Röhrchen mit einer nicht aktivierenden Menge eines Toll-like-Rezeptor-Liganden und einem CCR5-Liganden, vorzugsweise RANTES oder MIP-1α, und Material zum Nachweis der Konzentration eines der Zytokine TNF-α, IL-6, IL-8 und IFNγ, und/oder
- mindestens ein Röhrchen mit einem Toll-like-Rezeptor-Liganden und einem 2- oder 3-wertigen Kation bevorzugt in Form eines löslichen Salzes von Calcium, Aluminium, Gadolinium, Strontium oder Magnesium und Material zum Nachweis von IL-1β.

10. Verwendung eines diagnostischen Kits, wie in einem der Ansprüche 5 bis 9 definiert zur prognostischen Einschätzung des Krankheitsverlaufs der rheumatoiden Arthritis unter Therapie, insbesondere unter Therapie mit einem TNF-inhibierenden Arzneimittel, zur Diagnose der rheumatoiden Arthritis und/oder zur prognostischen Einschätzung zur Schwere einer Gelenksdestruktion im Rahmen der rheumatoiden Arthritis, vorzugsweise in einem Verfahren nach einem der Ansprüche 1 bis 4.

## Claims

1. Method for prognostic evaluation of the disease progression of rheumatoid arthritis and/or diagnosis of rheumatoid arthritis by analysing cytokines from a human full blood sample, wherein
- a volume of an isolated full blood sample of a human is transferred into at least one test tube containing a stimulating agent, whereas the stimulating agent is a specific binding partner of tmTNFα, a ligand of CCR5 or a divalent or trivalent cation, and as control samples, the same volume of the full blood sample in each case is transferred into an empty test tube as a negative control and a test tube containing lipopolysaccharide as a positive control respectively,
- the respective test tubes along with the full blood samples contained therein are subsequently incubated at a temperature of 30 - 40 °C,
- the cell-free residue of each full blood sample is obtained from the test tubes and the concentration of at least two cytokines selected from TNF-α, IL-8, IFNγ, IL-10, IL-1β and IL-6 in the cell-free residue of each sample is detected,
- by way of an altered concentration of the at least one cytokine in the at least one test tube comprising the stimulating agent, the prognostic evaluation of the disease progression of rheumatoid arthritis and/or the diagnosis of rheumatoid arthritis is subsequently made in comparison with the negative control,
- whereby a higher concentration of TNF-α, IL-8, IFNγ and IL-6 in the sample with the specific binding partner of tmTNF-α in comparison to the negative control indicate a higher risk that rheumatoid arthritis is present,
whereby a lower concentration of IL-1β or IL-10 in the sample with the specific binding partner of tmTNF-α in comparison to the negative control indicates that rheumatoid arthritis is present,
whereby in course of an a TNF inhibitor treatment a lower concentration of IL-1β in the sample with the specific binding partner of tmTNF-α in comparison to the negative control indicates a poor response to a TNF inhibitor treatment and a higher concentration of TNF-α or IL-8 in the sample with the specific binding partner of tmTNF-α in comparison to the negative control indicates a good response to a TNF inhibitor treatment,
whereby a lower concentration of TNF-α, IL-6, IL-8 and IFNγ in the sample with the ligand of CCR5 in comparison with the standard values of healthy individuals without the genetic deletion of CCR5 indicates lower risk that rheumatoid arthritis is present or in patients with a diagnosed rheumatoid arthritis indicates a good prognosis of the disease progression,
whereby a higher concentration of IL-1β in the sample with the divalent or trivalent cation in comparison to the negative control indicates a higher risk that rheumatoid arthritis is present.

2. Method according to any of the preceding claims, wherein a non-activating concentration of a toll-like receptor ligand is additionally mixed with the full blood sample in the at least one test tube comprising the stimulating agent.

3. Method according to any of the preceding claims, wherein the specific binding partner of tmTNF-α is anti-TNF-α or TNFR2:Ig.

4. Method according to any of the preceding claims, wherein the ligand of CCR5 is RANTES or MIP-1α.

5. Use of a diagnostic kit comprising:
- at least one test tube, containing:
∘ at least one stimulating agent, selected from a specific binding partner of tmTNF-α, preferably anti-TNF-α or TNFR2:Ig, a CCR5 ligand, preferably RANTES or MIP-1α, and/or a divalent or trivalent cation, preferably of the metals calcium, aluminium, gadolinium, strontium or magnesium, preferably in the form of a soluble salt,
∘ at least one toll-like receptor ligand,
- at least two comparison test tubes, of which a first test tube is an untreated empty test tube for the negative control and a second test tube for the positive control comprises bacterial lipopolysaccharide,
- optionally means for determining the cytokine concentration.
for diagnosis or prognostic evaluation of the disease progression of rheumatoid arthritis.

6. Use according to claim 5, wherein the test tube comprising the stimulating agent contains a non-activating amount of a toll-like receptor ligand.

7. Use according to one of the claims 5 or 6, comprising at least one test tube comprising a specific binding partner of tmTNF-α, preferably anti-TNF-α or TNFR2:Ig and/or at least one test tube comprising a CCR5 ligand, preferably RANTES or MIP-1α and/or at least one test tube comprising a divalent or trivalent cation, preferably in the form of a soluble salt of calcium, aluminium, gadolinium, strontium or magnesium.

8. Kit according to any of claims 5 to 7, wherein the test tube comprising the stimulating agent contains a specific binding partner of tmTNF-α, preferably anti-TNF-α or TNFR2:lg in a surface-bonded form.

9. Use according to any of claims 5 to 8, comprising, as the test tube comprising a stimulating agent,
- at least one test tube comprising a non-activating amount of a toll-like receptor ligand and a specific binding partner of tmTNF-α, preferably anti-TNF-α or TNFR2:lg, and equipment for detecting the concentration of one of the cytokines TNF-α, IL-8, IFNγ, IL-10 and IL-β,
- at least one test tube comprising a non-activating amount of a toll-like receptor ligand and a CCR5 ligand, preferably RANTES or MIP-1α, and equipment for detecting the concentration of one of the cytokines TNF-α, IL-6, IL-8 and IFNγ, and/or
- at least one test tube comprising a toll-like receptor ligand and a divalent or trivalent cation, preferably in the form of a soluble salt of calcium, aluminium, gadolinium, strontium or magnesium, and equipment for detecting IL-1β.

10. Use of a diagnostic kit as defined in any of claims 5 to 9 for prognostic evaluation of the disease progression of rheumatoid arthritis, for prognostic evaluation of the disease progression during treatment, in particular during treatment using a TNF-inhibiting drug, for diagnosis of rheumatoid arthritis and/or for prognostic evaluation of the severity of joint destruction in relation to rheumatoid arthritis, preferably by a method according to any of claims 1 to 4.

## Revendications

1. Procédé d'estimation du pronostic de l'évolution de la maladie pour l'arthrite rhumatoïde et/ou de diagnostic de l'arthrite rhumatoïde par analyse des cytokines provenant d'un échantillon de sang entier humain, dans lequel
- un volume d'un échantillon de sang entier humain isolé est transféré dans au moins un petit tube contenant un agent stimulant, dans lequel l'agent stimulant est un partenaire de liaison spécifique de tmTNF-α, un ligand de CCR5 ou un cation bivalent ou trivalent, et, faisant office d'échantillons témoins, le même volume de l'échantillon de sang entier est transféré dans respectivement un petit tube vide en tant que contrôle négatif et dans un tube contenant un lipopolysaccharide en tant que contrôle positif,
- les petits tubes respectifs sont ensuite incubés avec les échantillons de sang entiers contenus dedans, à une température de 30 - 40 °C,
- l'on obtient le surnageant acellulaire de l'échantillon de sang entier à partir des petits tubes et la concentration d'au moins deux cytokines sélectionnées parmi TNF-α, IL-8, IFNγ, IL-10, IL-1β et IL-6 est calculée dans le surnageant acellulaire de chaque échantillon,
- l'estimation du pronostic de l'évolution de la maladie de l'arthrite rhumatoïde et/ou le diagnostic de l'arthrite rhumatoïde est effectuée à l'aide d'une concentration modifiée d'au moins une cytokine dans l'au moins un petit tube comportant l'agent stimulant, par comparaison avec le contrôle négatif,
- dans lequel une concentration accrue en TNF-α, IL-8, IFNγ et IL-6 dans l'échantillon comportant le partenaire de liaison spécifique de tmTNF-a par comparaison au contrôle négatif indique un risque accru de l'existence d'une arthrite rhumatoïde,
dans lequel une concentration moindre de IL-1β ou de IL-10 dans l'échantillon comportant le partenaire de liaison spécifique de tmTNF-α par comparaison au contrôle négatif indique la présence d'une arthrite rhumatoïde,
dans lequel, lors d'une thérapie par inhibiteur de TNF, une concentration moindre de IL-1β dans l'échantillon comportant le partenaire de liaison spécifique de tmTNF-α par comparaison au contrôle négatif indique une mauvaise réponse à la thérapie et une concentration accrue de TNF-α ou IL-8 dans l'échantillon comportant le partenaire de liaison spécifique de tmTNF-α par comparaison au contrôle négatif indique une bonne réponse à la thérapie,
dans lequel une concentration moindre de TNF-α, IL-6, IL-8 et IFNγ dans l'échantillon comportant le ligand de CCR5 en comparaison à la valeur normale des individus sans la délétion génétique de CCR5 indique un risque faible de l'existence d'une arthrite rhumatoïde ou, chez des patients atteints d'une arthrite rhumatoïde avérée, indique un bon pronostic pour l'évolution de leur affection,
dans lequel une concentration accrue de IL-1β dans l'échantillon comportant le cation bivalent ou trivalent en comparaison du contrôle négatif indique un risque accru de l'existence d'une arthrite rhumatoïde.

2. Procédé selon l'une des revendications précédentes, dans lequel l'échantillon de sang entier est déplacé dans l'au moins un petit tube contenant l'agent stimulant, de plus contenant une concentration non activante d'un ligand récepteur de type Toll.

3. Procédé selon l'une des revendications précédentes, dans lequel le partenaire de liaison spécifique de tmTNF-α est anti-TNF-α ou TNFR2:Ig.

4. Procédé selon l'une des revendications précédentes, dans lequel le ligand CCR5 est RANTES ou MIP-1α.

5. Utilisation d'une trousse de diagnostic comprenant :
- au moins un petit tube contenant
∘ au moins un agent stimulant sélectionné parmi un partenaire de liaison spécifique de tmTNF-α, de préférence anti-TNF-α ou TNFR2:Ig, un ligand CCR5, de préférence RANTES ou MIP-1α, et/ou un cation bivalent ou trivalent, de manière préférée des métaux calcium, aluminium, gadolinium, strontium ou magnésium, de manière préférée sous la forme d'un sel soluble,
∘ au moins un ligand récepteur de type Toll,
- au moins deux petits tubes témoins, parmi lesquels un premier petit tube est un petit tube vide non traité pour le contrôle négatif et un second petit tube contient un lipopolysaccharide bactérien pour le contrôle positif,
- le cas échéant un moyen de déterminer la concentration en cytokine,
pour le diagnostic ou l'estimation du pronostic de l'évolution de la maladie de l'arthrite rhumatoïde.

6. Utilisation selon la revendication 5, dans laquelle le petit tube comportant l'agent stimulant contient une quantité non activante d'un ligand récepteur de type Toll.

7. Utilisation selon l'une des revendications 5 ou 6, comprenant au moins un petit tube comportant un partenaire de liaison spécifique de tmTNF-α, de préférence anti-TNF-α ou TNFR2:Ig et/ou au moins un petit tube comportant un ligand CCR5, de préférence RANTES ou MIP-1α et/ou au moins un petit tube comportant un cation bivalent ou trivalent de manière préférée sous la forme d'un sel soluble de calcium, d'aluminium, de gadolinium, de strontium ou de magnésium.

8. Utilisation selon l'une des revendications 5 à 7, dans lequel le petit tube comportant l'agent stimulant contient un partenaire de liaison spécifique de tm-TNF-α, de préférence anti-TNF-α ou TNFR2:Ig sous une forme liée à une surface.

9. Utilisation selon l'une quelconque des revendications 5 à 8, comprenant, en tant que petit tube comportant un agent stimulant :
- au moins un petit tube comportant une quantité non activante d'un ligand récepteur de type Toll et un partenaire de liaison spécifique de tmTNF-α, de préférence anti-TNF-α ou TNFR2:Ig, et du matériel de mise en évidence de la concentration d'une des cytokines TNF-α, IL-8, IFNγ, IL-10 et IL-1β,
- au moins un petit tube comportant une quantité non activante d'un ligand récepteur de type Toll et un ligand CCR5, de préférence RANTES ou MIP-1α, et du matériel de mise en évidence de la concentration d'une des cytokines TNF-α, IL-6, IL-8 et IFNγ, et/ou
- au moins un petit tube comportant un ligand récepteur de type Toll et un cation bivalent ou trivalent de préférence sous la forme d'un sel soluble de calcium, d'aluminium, de gadolinium, de strontium ou de magnésium et du matériel de mise en évidence de IL-1β.

10. Utilisation d'une trousse de diagnostic, telle que définie dans l'une des revendications 5 à 9, pour l'estimation du pronostic de l'évolution de la maladie de l'arthrite rhumatoïde faisant l'objet d'une thérapie, en particulier une thérapie avec des médicaments inhibiteurs de TNF, pour le diagnostic de l'arthrite rhumatoïde et/ou pour l'estimation du pronostic de la gravité d'une destruction articulaire dans le cadre d'une arthrite rhumatoïde, de préférence dans un procédé selon l'une des revendications 1 à 4.
